# EUROPEAN PATENT SPECIFICATION

(11) **EP 4 235 683 B1**
(45) Date of publication and mention of the grant of the patent: **22.10.2025**
(21) Application number: 21882924.0
(22) Date of filing: 22.10.2021
(51) Int. Cl.: G16H 20/00, G16H 50/20, G16H 50/30, G16H 50/50, G16H 50/70, A61B 5/00

(54) **HEALTH IMPROVEMENT PATH SEARCH DEVICE AND HEALTH IMPROVEMENT PATH SEARCH METHOD**
VORRICHTUNG ZUR SUCHE NACH GESUNDHEITSVERBESSERUNGSPFADEN UND VERFAHREN ZUR SUCHE NACH GESUNDHEITSVERBESSERUNGSPFADEN
DISPOSITIF DE RECHERCHE DE VOIE D'AMÉLIORATION DE SANTÉ ET PROCÉDÉ DE RECHERCHE DE VOIE D'AMÉLIORATION DE SANTÉ

(30) Priority: 23.10.2020 JP 2020178148
(43) Date of publication of application: 30.08.2023
(73) Proprietor: Kyowa Hakko Bio Co., Ltd., Tokyo 100-8185 (JP); Kyoto University, Kyoto-shi, Kyoto 606-8501 (JP); HIROSAKI UNIVERSITY, Hirosaki-shi, Aomori 036-8560 (JP)
(72) Inventor: NAKAMURA, Kazuki, Tokyo 100-8185 (JP); OKUNO, Yasushi, Kyoto-shi, Kyoto 606-8501 (JP); KOJIMA, Ryosuke, Kyoto-shi, Kyoto 606-8501 (JP); UCHINO, Eiichiro, Kyoto-shi, Kyoto 606-8501 (JP); MURASHITA, Koichi, Hirosaki-shi, Aomori 036-8560 (JP); ITOH, Ken, Hirosaki-shi, Aomori 036-8560 (JP); NAKAJI, Shigeyuki, Hirosaki-shi, Aomori 036-8560 (JP)
(74) Representative: Vossius & Partner Patentanwälte Rechtsanwälte mbB
(86) International application number: PCT/JP2021/039081
(87) International publication number: WO 2022/085785

(56) References cited:
- EP-A1- 3 624 133
- CN-A- 108 717 863
- JP-A- 2009 211 126
- JP-A- 2018 055 424
- JP-A- 2020 003 882
- JP-A- 2020 042 761
- US-A1- 2014 058 738
- US-A1- 2014 058 738
- US-A1- 2018 089 385
- KAZUKI NAKAMURA ET AL: "Health improvement framework for planning actionable treatment process using surrogate Bayesian model", ARXIV.ORG, CORNELL UNIVERSITY LIBRARY, 201 OLIN LIBRARY CORNELL UNIVERSITY ITHACA, NY 14853, 30 October 2020 (2020-10-30), XP081802912

## Description

### Technical Field

One aspect of the present invention relates to a health improvement path search device and a health improvement path search method.

### Background Art

Personalized medical care is expected to be able to be a treatment method for performing medical decisions, treatments, or interventions matching an individual's physical constitution, environment, and the like. As an example of a technology relating to individual medical care, in Patent Literature 1, a lifestyle habit improvement assistance system that extracts an improvement factor that is a factor to be improved in lifestyle habit information on the basis of biological information of a user and determines an improvement plan for improving the improvement factor is disclosed.
CN 108 717 863 A relates to a system comprises an electric cooker, a cloud and an application program installed on terminal equipment. The electric cooker obtains information of food put into the electric cooker by a user and sends the food information to the cloud. The cloud is used for receiving the food information sent by the electric cooker, determining a diet plan of the user according to the food information and sending the diet plan of the user to the application program. The application program is used for receiving the diet plan sent by the cloud and pushing the diet plan to the user.

### Citation List

### Patent Literature

[Patent Literature 1] Japanese Unexamined Patent Publication No. 2009-217703

### Non-Patent Literature

[Non-Patent Literature 1] T. Chen, C. Guestrin, XGBoost, in: Proceedings of the 22nd ACM SIGKDD International Conference on Knowledge Discovery and Data Mining, ACM, New York, NY, USA, 2016: pp. 785-794.
[Non-Patent Literature 2] Tibshirani, R., Johnstone, I.,Hastie, T. & Efron, B. Least angle regression. The Annals of Statistics 32,407-499 (2004).
[Non-Patent Literature 3] Hastie, T. & Efron, B. lars:Least Angle Regression, Lasso and Forward Stagewise. R package version 1.2 (2013).

### Summary of Invention

### Technical Problem

Here, although the invention disclosed in Patent Literature 1 can propose an improvement plan for lifestyle habits, a specific improvement process is not proposed thereby, and thus, an improvement process that can be performed (in other words, can be easily handled) by a person is not proposed.

One aspect of the present invention is in consideration of the situations described above, and an object thereof is to propose a health improvement process that can be performed by a person.

### Solution to Problem

According to one aspect of the present invention, there is provided a health improvement path search device comprising: a first model generating unit (12) configured to generate a first model predicting a value of a health index that is an objective variable on the basis of values of a plurality of explanatory variables; a second model generating unit (13) configured to generate a second model deriving a probability of taking a state calculated using the first model representing a degree of easiness of presence of each of combinations of the values of the plurality of explanatory variables input to the first model and the value of the health index predicted using the first model on the basis of the values of the plurality of explanatory variables; and a path searching unit (14) configured to divide a variable space of the plurality of explanatory variables into a lattice, and in a graph with lattice points as nodes, to derive the value of the health index and the probability corresponding to each node on the basis of the first model and the second model with a plurality of nodes within a predetermined range from current values of the plurality of explanatory variables set as inputs, specify a plurality of paths transitioning to each of the nodes from the current values as start points for transitioning between the values of the plurality of explanatory variables of continuous values, specify one or a plurality of paths in which the value of the health index at an end point is improved from the current value of the health index among the plurality of paths as candidate paths, and specify a path for which a product of the probabilities of the nodes included in the path is a maximum among the candidate paths as a health improvement path; wherein variables refer to measured values of a person measured in a health examination or the like, explanatory variables are variables that become causes of a causal relationship, and objective variables are variables that become a result of the causal relationship; and wherein the health improvement path is an improvement sequence for measurement values of a person that is performed for improving values of health indexes.

According to one aspect of the present invention, there is provided a health improvement path search method performed by an information processing device, the health improvement path search method comprising: a step of generating a first model (S1) predicting a value of a health index that is an objective variable on the basis of values of a plurality of explanatory variables; a step of generating a second model (S2) deriving a probability of taking a state calculated using the first model representing a degree of easiness of presence of each of combinations of the values of the plurality of explanatory variables input to the first model and the value of the health index predicted using the first model on the basis of the values of the plurality of explanatory variables; and a step (S3) of divide a variable space of the plurality of explanatory variables into a lattice, and in a graph with lattice points as nodes, and deriving the value of the health index and the probability corresponding to node on the basis of the first model and the second model with a plurality of nodes within a predetermined range from current values of the plurality of explanatory variables set as inputs, specifying a plurality of paths transitioning to each of the measurement target values from the current values as start points for transitioning between the values of the plurality of explanatory variables of continuous values, specifying one or a plurality of paths in which the value of the health index at an end point is improved from the current value of the health index among the plurality of paths as candidate paths, and specifying a path for which a product of the probabilities of the nodes included in the path is a maximum among the candidate paths as a health improvement path; wherein variables refer to measured values of a person measured in a health examination or the like, explanatory variables are variables that become causes of a causal relationship, and objective variables are variables that become a result of the causal relationship; and wherein the health improvement path is an improvement sequence for measurement values of a person that is performed for improving values of health indexes.

In the health improvement path search device and the health improvement path search method according to one aspect of the present invention, a first model predicting a health index that is an objective variable is generated on the basis of explanatory variables, and a second model deriving a presence probability of each of combinations of a plurality of input values of the plurality of explanatory variables and a value of a health index that is a predicted value thereof is generated. When a plurality of measurement target values are input to the first model and the second model, a presence probability for each combination of each measurement target value and a value of the health index that is a predicted value thereof is derived. Then, in this health improvement path search device and this health improvement path search method, among a plurality of paths transitioning from current values of the plurality of explanatory variables as a start point to each measurement target value, a path for which the value of the health index at an end point is improved from the current value of the health index, and a product of presence probabilities of the measurement target values within the path is a maximum is specified as a health improvement path. According to such a configuration, when current values of a plurality of explanatory variables acquired through a health examination or the like are input, from a plurality of measurement target values within a predetermined range from a current value, a presence probability of a combination of each measurement target value and a value of a health index that is a predicted value thereof is derived. Then, among paths transitioning between the measurement target values, a path for which a health index is improved from a start point to an end point, and a presence probability of each measurement target value within the path is a maximum is specified. The path specified in this way becomes a path that represents a sequence for going by way of only realistic values for transition to each measurement target value having a high presence probability until the health index is improved and specifically improves the health. By illustrating such a health improvement path, a health improvement process that can be performed by a person can be proposed.

### Advantageous Effects of Invention

According to one aspect of the present invention, a health improvement process that can be performed by a person, more specifically, an improvement sequence for measurement values of a person that can be performed for improving values of health indexes can be proposed.

### Brief Description of Drawings

FIG. 1 is a block diagram illustrating a functional configuration of a health improvement path search device.
FIG. 2 is a conceptual diagram illustrating a relation between explanatory variables and an objective variable in a prediction model.
FIG. 3 is a conceptual diagram illustrating a presence probability of data in a surrogate model.
FIG. 4 is a conceptual diagram illustrating a path search result.
FIG. 5 is a diagram illustrating pseudo codes of a path search algorithm.
FIG. 6 is a hardware configuration diagram of a health improvement path search device.
FIG. 7 is a flowchart of a process performed by a health improvement path search device.
FIG. 8 illustrates an example of a data set in Example 1.
FIG. 9 is a diagram illustrating a score of a regression model in Example 1.
FIG. 10 is a diagram illustrating a variable importance level of a regression model in Example 1.
FIG. 11 is a diagram illustrating a graphical model of a hierarchical Bayesian model in Example 1.
FIG. 12 is a diagram illustrating an evaluation result of WBIC in Example 1.
FIG. 13 is a histogram illustrating an actionability score of each instance in Example 1.
FIG. 14 is a diagram illustrating an example of a path search result in Example 1.
FIG. 15 illustrates an example of a data set in Example 2.
FIG. 16 is a diagram illustrating variable importance levels of a regression model in Example 2.
FIG. 17 is a diagram illustrating a score of a regression model in Example 2.
FIG. 18 is a diagram illustrating an evaluation result of WBIC in Example 2.
FIG. 19 is a histogram illustrating an actionability score of each instance in Example 2.
FIG. 20 is a diagram illustrating an example of a path search result in Example 2.

### Description of Embodiments

Hereinafter, embodiments will be described in detail with reference to the drawings. In description, the same reference signs will be assigned to the same element or elements having the same function, and duplicate description will be omitted.

A health improvement path search device 1 according to an embodiment is a device that predicts a health index that is an objective variable on the basis of a plurality of explanatory variables and searches for a path that becomes an improvement process of the health index. Variables refer to measured values of a person measured in a health examination or the like, in other words, physical characteristics, a body constitution, biological information, and the like, and more specifically, examples thereof include age, gender, a body height, a body mass, a BMI, a blood pressure level, a blood sugar level, HbAlc, γ-GTP, AST, ALT, albumin, creatinine, HDL cholesterol, LDL cholesterol, neutral fat, a blood oxygen saturation level, a lung capacity, red blood cells, white blood cells, a hematocrit value, a leg score, and the like. The explanatory variables are variables that become causes of a causal relationship, and the objective variable is a variable that becomes a result of the causal relationship. For example, measured values that become indexes of diseases and organ functions that are values of health indexes, more specifically, a blood pressure value that is an index of hypertension, a blood sugar level and HbA1c that are indexes of diabetes, creatinine that is an index of a kidney function, γ-GTP, AST, or ALT that is an index of liver function, HDL cholesterol, LDL cholesterol, or neutral fat that is an index of hyperlipidemia and arteriosclerosis, a blood oxygen saturation level and a lung capacity that are indexes of a lung function, and the like may be set as objective variables, and some or all of variables other than the objective variables may be set as explanatory variables. The path is a path joining a value of a current health index (a start point) to a value of an improved health index (an end point) for a health index predicted from a plurality of explanatory variables and represents an improvement sequence (an improvement process) of each variable. Here, in a case in which the path is set as a simply linear path (a shortest distance), there is a possibility of unrealistic values of variables that cannot be taken by a person being included on the path. In a case in which a path includes such an unrealistic value of a variable, this path is not a path that can be executed by a person. The health improvement path search device 1 avoids a path including unrealistic values of variables that cannot be taken by a person by searching for a path that runs stepwise by way of realistic values of variables that can be taken by a person.

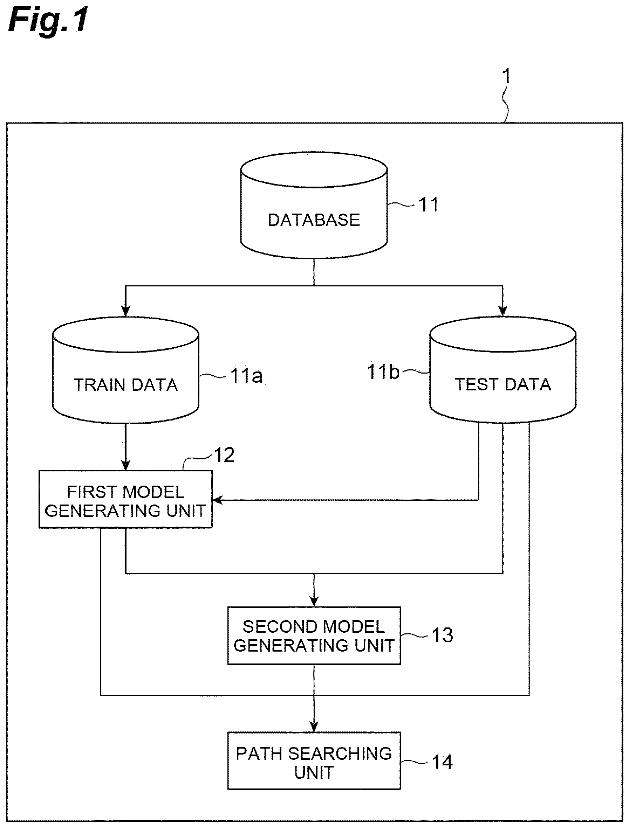

FIG. 1 is a block diagram illustrating a functional configuration of the health improvement path search device 1 according to this embodiment. The health improvement path search device 1 includes a database 11, a first model generating unit 12, a second model generating unit 13, and a path searching unit 14.

The database 11 stores data such as physical characteristics, a body constitution, biological information, and the like as a data set for each instance of a medical examinee of a health examination, a patient, or the like. For example, the data includes age, gender, a body height, a body mass, a blood pressure value, a blood sugar level, γ-glutamyl transpeptidase (γ-GTP), a leg score, and the like that are variables but is not limited thereto. The database 11 may be an external storage device that is accessible through a network such as the Internet or the like.

The first model generating unit 12 generates a first model predicting a value of a health index that is an objective variable on the basis of values of a plurality of explanatory variables. The first model generating unit 12 acquires a data set from the database 11 and performs machine learning, thereby generating a prediction model (a first model). For example, the first model generating unit 12 selects data of a data set as explanatory variables and objective variables and generates a regression model regressing a health index that is an objective variable from a plurality of explanatory variables. For example, the first model generating unit 12 generates a regression model regressing a blood pressure value that is an objective variable using body constitution data, blood data, and the like of each person as explanatory variables.

The first model generating unit 12 divides a data set acquired from the database 11 into train data 11a (a training data set) and test data 11b (a test data set) as preprocessing of machine learning. The first model generating unit 12 may randomly divide a data set such that the train data 11a and the test data 11b are at a predetermined ratio (for example, 80% and 20%). The first model generating unit 12 may standardize explanatory variables of continuous values using a mean (average) and a standard deviation. The first model generating unit 12 may substitute explanatory variables of discrete values with dummy variables using one-hot encoding. The first model generating unit 12 may complement missing values of explanatory variables using multiple imputation. The first model generating unit 12 may generate a regression model using XGBoost (see Non-Patent Literature 1) that is an algorithm of a Gradient Boosting Decision Tree (GBDT) system. The first model generating unit 12 may determine hyper parameters of a regression model using 5-fold cross validation for the train data 11a.

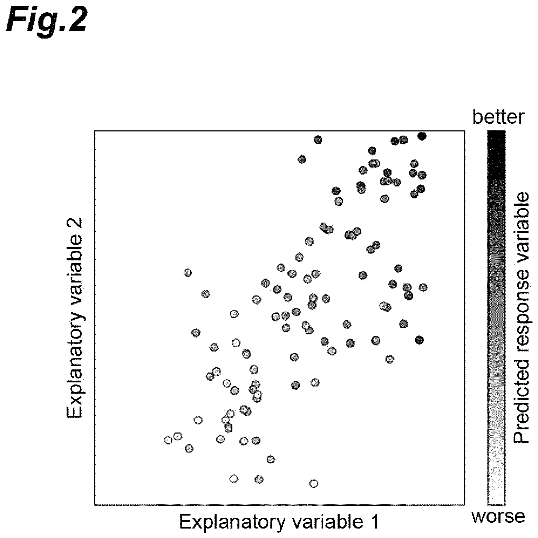

FIG. 2 is a conceptual diagram illustrating a relation between explanatory variables and an objective variable in a prediction model. In FIG. 2, a horizontal axis represents a first explanatory variable, a vertical axis represents a second explanatory variable, and a plot inside a graph that is a variable space represents an objective variable. The value of the objective variable regresses in accordance with a value of the first explanatory variable and a value of the second explanatory variable. Shading of the plot represents a degree of improvement of a health index of the objective variable. For example, the first explanatory variable and the second explanatory variable are body constitution data, blood data, and the like. For example, the objective variable is a value such as a blood pressure value or the like that is an improvement target.

Referring back to FIG. 1, the second model generating unit 13 generates a second model that derives a presence probability representing a degree of easiness of presence for each combination of values of a plurality of explanatory variables input to the first model and a value of the health index predicted on the basis of the values of the plurality of explanatory variables using the first model. For example, the second model generating unit 13 generates a surrogate model (a second model) that can calculate a degree of easiness of taking a prediction value in a variable space of the plurality of explanatory variables as a probability. For example, the second model generating unit 13 generates a hierarchical Bayesian model using hierarchical Bayesian modelling. The hierarchical Bayesian model can flexibly represent a probability even in a case in which input data is changed.

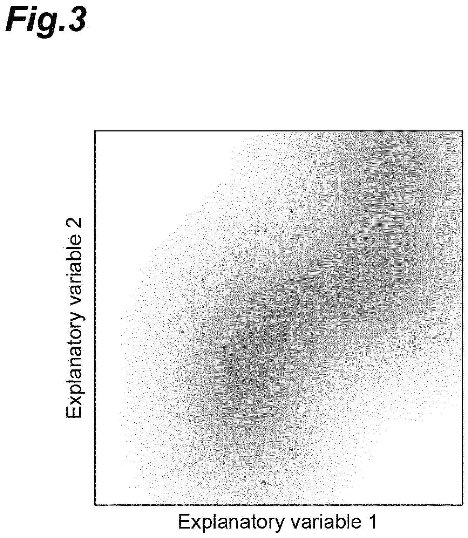

FIG. 3 is a conceptual diagram illustrating a presence probability of data in a surrogate model. In FIG. 3, a horizontal axis represents a first explanatory variable, a vertical axis represents a second explanatory variable, and shading inside a graph that is a variable space represents a presence probability of data. FIG. 3 illustrates a relation between values of the first explanatory variable and the second explanatory variable and a presence probability of a combination of values of health indexes that are prediction values. In FIG. 3, the higher the presence probability of data, the darker the shading of the inside of the graph.

Referring back to FIG. 1, the path searching unit 14 derives a value of health index and a presence probability corresponding to each measurement target value on the basis of the first model and the second model with a plurality of measurement target values inside a predetermined range from current values of the plurality of explanatory variables set as inputs, specifies a plurality of paths transitioning between measurement target values with a current value set as a start point for transitioning between values of a plurality of explanatory variables that are continuous to each other, specifies one or a plurality of paths in which the value of a health index of an end point is improved from a current value of the health index as candidate paths among a plurality of paths, and specifies a path in which a product of presence probabilities of measurement target values included in the path is a maximum among candidate paths as a health improvement path.

The path searching unit 14, for example, predicts objective variables such as a blood pressure value and the like from explanatory variables such as a body mass, blood data, and the like using a prediction model and derives a degree of easiness of taking the value as a presence probability using a surrogate model. The path searching unit 14, by changing values of a plurality of explanatory variables within a predetermined range and setting the values as inputs to the prediction model and the surrogate model, may set inputs of the measurement target values. The path searching unit 14 derives a value of a health index corresponding to each input measurement target value and a presence probability, specifies a path transitioning each measurement target value with the current value set as a start point, and specifies a path in which a product of presence probabilities of the measurement target values included in the path is a maximum among candidate paths in which a value of a health index at an end point is improved from the current value of the health index as a health improvement path.

In addition, although all the explanatory variables may intervene in the prediction model, there are explanatory variables that are not appropriate for a path search (for example, gender and the like). Thus, in performing a path search, it is necessary to select appropriate explanatory variables. The path searching unit 14 determines explanatory variables intervening in the prediction model (hereinafter, referred to as "intervention variables") among all the explanatory variables. For example, the path searching unit 14 may determine intervention variables by selecting variables of a predetermined number from high rankers of importance of variables in the prediction model or the like. The path searching unit 14 predicts objective variables using a prediction model by using the determined intervention variables.

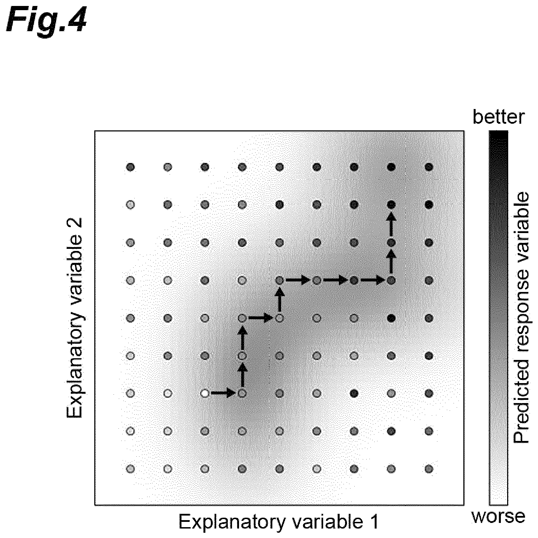

The path searching unit 14 handles a variable space of a plurality of explanatory variables as a graph partitioned in a lattice shape and joining lattice points as nodes, thereby building a path. In this specification, a probability of taking a state of each node calculated using the prediction model and the surrogate model is defined as a probability of the node, and a product of probabilities of nodes on a specific path is defined as "actionability". The larger the value of the actionability, nodes having high presence probabilities are gone through, and it represents that the path can be executed by a person. On the other hand, the smaller the value of the actionability, nodes having low presence actionabilities are gone through, and it represents that the path cannot be executed by a person. The path searching unit 14, for example, calculates a negative value of logarithm of actionability as a path cost and acquires a path in which a path cost to each node corresponding to each measurement target value is a minimum using a node corresponding to a current value as a start point. In other words, the path searching unit 14 acquires a path for which a path cost to each node is a minimum, and the actionability is a maximum. A conceptual diagram of the path acquired in this way is illustrated in FIG. 4. FIG. 4 illustrates an example of a sequence of a path using arrows between nodes.

The path searching unit 14 may specify a path for which the value of the health index is improved the most among paths for which the value of the health index is improved from the current value of the health index as a candidate path. In addition, after a first process of selecting values of a plurality of explanatory variables that are approximated to a reference value as measurement target values with a current value set as the reference value is performed, the path searching unit 14 may repeatedly perform a second process of selecting values of a plurality of explanatory variables that are approximated to a reference value as measurement target values with a measurement target value of which a presence probability at the time of being input to the second model is the highest among the selected measurement target values set as the new reference value.

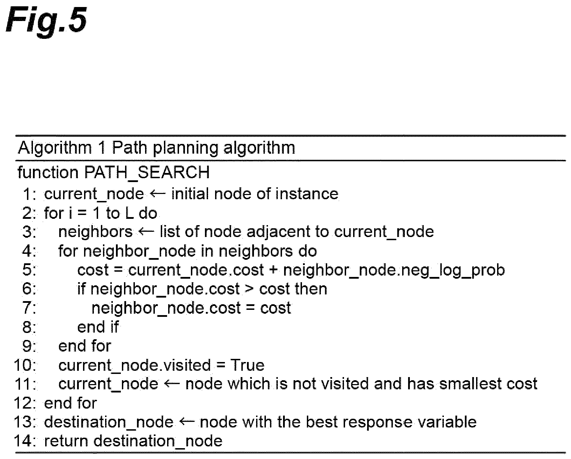

FIG. 5 is a diagram illustrating pseudo codes of a path search algorithm. The path searching unit 14 searches for a path to a node achieving a prediction value that is improved the most within a search repetition number L in a width priority search using the pseudo codes as illustrated in FIG. 5. The path searching unit 14 acquires a list of nodes adjacent to the current node in a third line of the pseudo codes and updates a path cost for such nodes in 5th to 7th lines. For example, the adjacent nodes are nodes acquired by changing a value of the current node by one unit (for example, 0.2σ of each intervention variable in the train data 11a). An adjacent node may be also referred to as an approximated node. One unit corresponds to a size of a cell acquired by partitioning a variable space into lattice shapes. The path searching unit 14 selects a node that becomes a next search start point in an 11th line. A node that becomes a search start point is a node for which a path cost is a minimum among nodes that have not been searched. The path searching unit 14 performs looping such that path searches of a predetermined number (for example, a search repetition number L = 20,000 times) is performed in 2nd to 12th lines, and, in a 13th line, selects a node of which a prediction value of a regression model is improved the most as an end point node and acquires a path to an end point node as a health improvement path. In a case in which there are a plurality of nodes of which prediction values are the same, the path searching unit 14 acquires a path for which the path cost is a minimum as a health improvement path.

The path searching unit 14 may specify a shortest path randomly transitioning between the measurement target values from a start point to an end point as a random path and specify a path in which a product of presence probabilities of measurement target values included in the path is a maximum and is equal to or larger than a product of presence probabilities of measurement target values included in the random path as a health improvement path among candidate paths. For example, the path searching unit 14 specifies an optimal path that is a path for which a product of presence probabilities of measurement target values included in the path is a maximum among candidate paths acquired using a path search algorithm. The path searching unit 14 specifies a path acquired by randomly joining a start point and an end point of the optimal path in a shortest sequence. Then, the path searching unit 14 specifies a path for which a product of presence probabilities of measurement target values included in the optimal path is equal to or larger than a product of presence probabilities of measurement target values included in the random path as a health improvement path.

For example, the path searching unit 14 calculates a score represented as actionability score = log (actionability of optimal path) - log (actionability of random path). Here, for example, a random path actionability may be a geometric mean of actionabilities of 10 random paths. The actionability score represents relative efficiency of the optimal path actionability with respect to the random path actionability. In a case in which the actionability score is 0, the optimal path has the same actionability as that of the random path. The higher the actionability score, the path searching unit 14 can evaluate that the actionability of the optimal path is higher than that of the random path. In a case in which the actionability score is lower than 0, the path searching unit 14 can evaluate that the actionability of the optimal path is higher than that of the random path. In this way, the path searching unit 14 evaluates validity of the optimal path.

The path searching unit 14 outputs result data that represents a search result of a path. A data structure of the result data is not particularly limited.

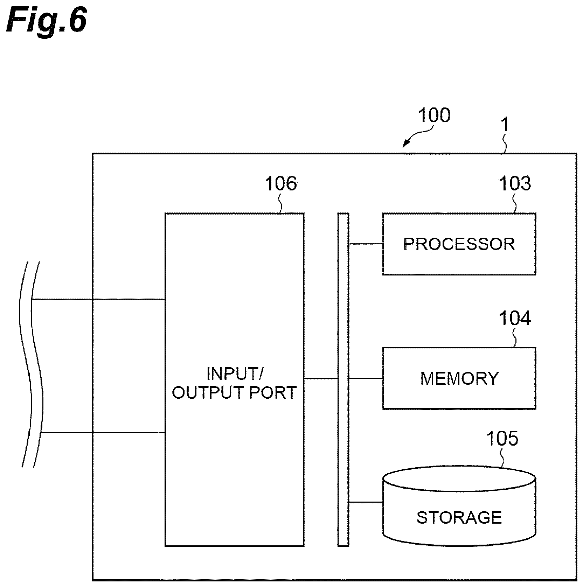

FIG. 6 is a hardware configuration diagram of the health improvement path search device 1. As illustrated in FIG. 6, the health improvement path search device 1 is configured using an information processing device 100 that includes one or a plurality of processors 103, a memory 104, a storage 105, and an input/output port 106. The input/output port 106 performs input/output of control signals to/from an external device and the like. The storage 105 stores a program for performing various processes. The storage 105 may be any storage as long as it is a computer-readable storage. Specific examples thereof include a hard disk, a nonvolatile semiconductor memory, a magnetic disk, an optical disc, and the like. The memory 104 temporarily stores a program loaded from the storage 105, arithmetic operation results of the processor 103, and the like. By executing a program in cooperation with the memory 104, the processor 103 configures each of the functional modules described above.

In addition, the hardware configuration of the health improvement path search device 1 is not limited to configuring of each functional module using a program. For example, each functional module of the health improvement path search device 1 may be configured using a dedicated logical circuit or an application specific integrated circuit (ASIC) acquired by integrating this.

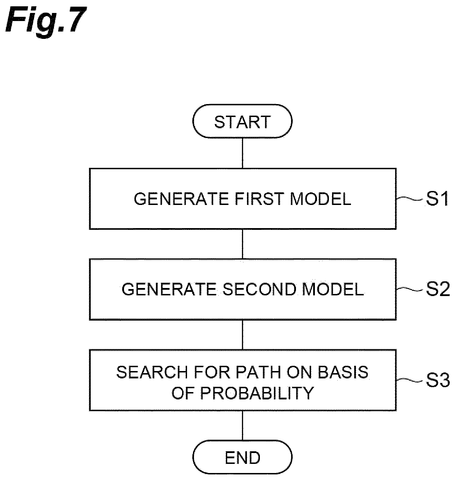

Next, a health improvement path search method performed by the health improvement path search device 1 will be described with reference to FIG. 7. FIG. 7 is a flowchart of a process performed by the health improvement path search device 1.

The health improvement path search device 1 generates a first model predicting a value of a health index that is an objective variable on the basis of values of a plurality of explanatory variables (Step S1). The health improvement path search device 1, for example, selects data of a data set as explanatory variables and an objective variable and generates a regression model regressing a health index that is an objective variable from a plurality of explanatory variables.

The health improvement path search device 1 generates a second model deriving a presence probability representing a degree of easiness of presence for each combination of values of a plurality of explanatory variables input to the first model and a value of the health index predicted on the basis of the values of the plurality of explanatory variables using the first model (Step S2). For example, the health improvement path search device 1 generates a hierarchical Bayesian model using hierarchical Bayesian modeling.

The health improvement path search device 1 derives a value of a health index and a presence probability corresponding to each measurement target value on the basis of the first model and the second model with a plurality of measurement target values within a predetermined range from current values of a plurality of explanatory variables set as inputs, specifies a plurality of paths transitioning to each measurement target value from a current value as a start point for transitioning between values of a plurality of explanatory variables that are continuous to each other, specifies one or a plurality of paths in which the value of the health index at an end point is improved from the current value of the health index among a plurality of paths as candidate paths, and specifies a path for which a product of presence probabilities of measurement target values included in the path is a maximum among candidate paths as a health improvement path (Step S3). For example, the health improvement path search device 1 predicts an objective variable using a prediction model by changing explanatory variables such as a body mass, blood data, and the like and derives a degree of easiness of taking the value as a presence probability using a surrogate model. The health improvement path search device 1 derives a value of a health index and a presence probability corresponding to each input measurement target value, specifies a path transitioning to each measurement target value with a current value set as a start point, and specifies a path for which a product of presence probabilities of measurement target values inside the path is a maximum among candidate paths in which a value of the health index at an end point is improved from the current value of the health index as a health improvement path.

Hereinafter, although examples will be described more specifically, the present disclosure is not limited thereto at all.

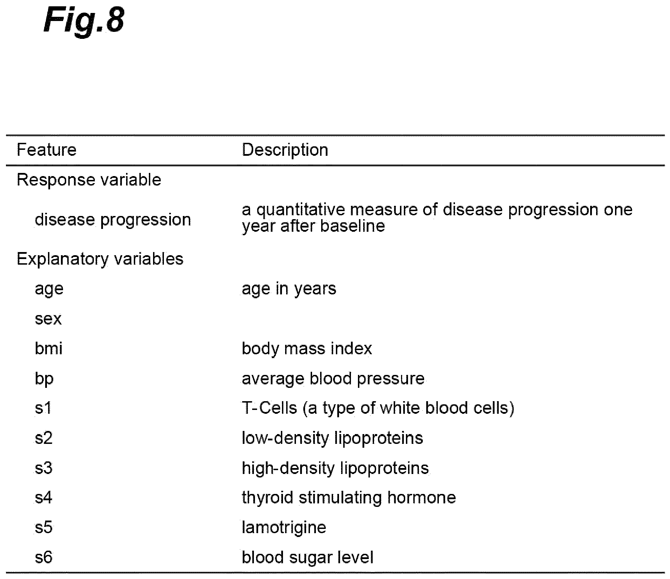

[Example 1] In Example 1, actionability is evaluated using a public data set relating to diabetes (see Non-Patent Literatures 2 and 3) (hereinafter, simply referred to as a "public data set") as a data set to be bench-marked. FIG. 8 illustrates an example of a data set in Example 1. Explanatory variables include age, gender, a bmi, an average blood pressure, T-Cells, low-density lipoproteins, high-density lipoproteins, thyroid stimulating hormone, lamotrigine, and a blood sugar level. Missing values are not included in the public data set.

In Example 1, by randomly dividing the public data set with a ratio of 80% and 20% and using respective divided parts as a training data set and a test data set, a regression model is generated using XGBoost. A regression model is a model that regresses a future progression degree of diabetes that is an objective variable from explanatory variables of continuous values of 9 types and an explanatory variable of a discrete value of one type in a public data set.

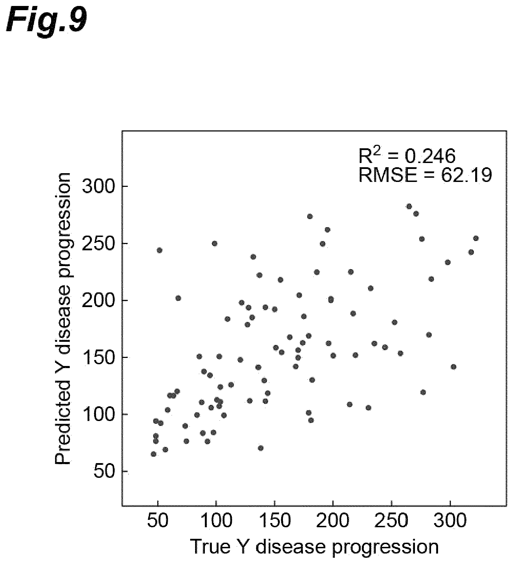

FIG. 9 is a diagram illustrating a score of a regression model in Example 1. In FIG. 9, a horizontal axis represents a true objective variable, and a vertical axis represents a prediction value of the objective variable using the regression model. In the generated regression model, Root Mean Squared Error (RMSE) of a test data set is 62.19, and R² (a determination coefficient) is 0.246.

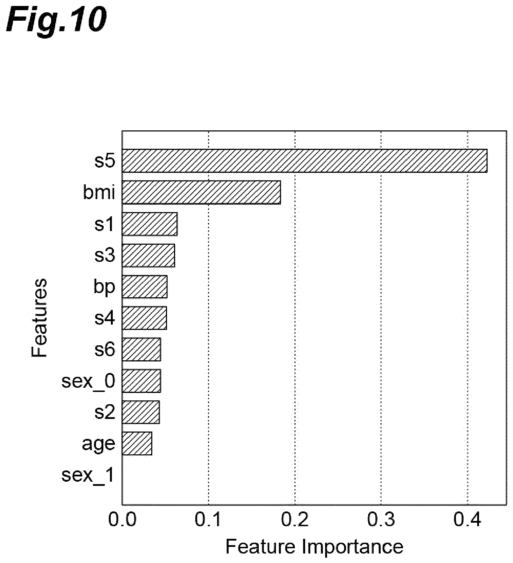

FIG. 10 is a diagram illustrating a variable importance level of a regression model in Example 1. In FIG. 10, a horizontal axis represents a variable importance level, and a vertical axis represents a type of variable. In a regression model using XGBoost, variable importance levels in the regression model can be calculated. A variable importance level may be also referred to as a degree of contribution in a regression model using XGBoost. In Example 1, as intervention variables in a path search, five high-ranking variables of the variable importance level are selected. More specifically, the five high-ranking variables are a bmi, a bp (a blood pressure value), s1 (T cells), s3 (high-density lipoproteins), and s5 (lamotrigine).

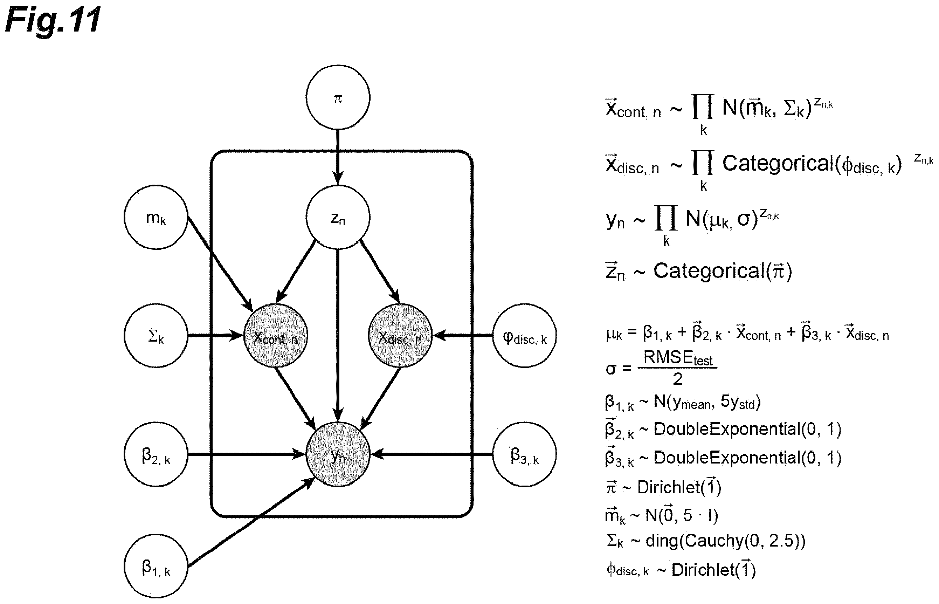

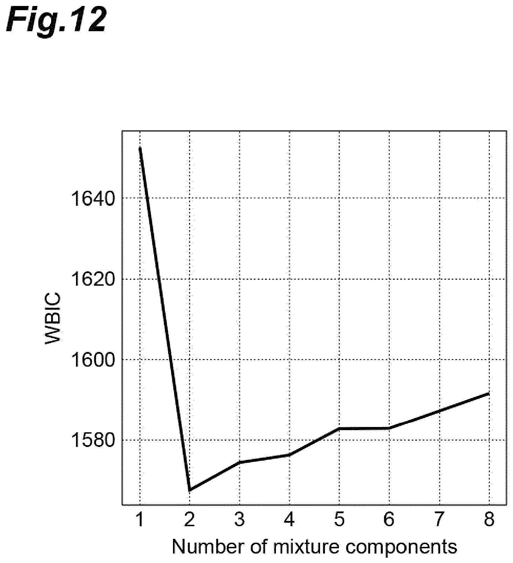

Next, a hierarchical Bayesian model is derived on the basis of the public data set and a prediction value of the regression model using hierarchical Bayesian modeling. FIG. 11 is a diagram illustrating an example of a graphical model of a hierarchical Bayesian model in Example 1. In Example 1, a valid number of mixture components in the hierarchical Bayesian model is evaluated using a Widely applicable Bayesian information criterion (WBIC). The mixture components can be also referred to as the number of cluster of data in a hierarchical Bayesian model. FIG. 12 is a diagram illustrating an evaluation result of WBIC in Example 1. In FIG. 12, a horizontal axis represents the number of mixture components, and a vertical axis represents a value of the WBIC. The smaller the value of the WBIC, the higher the validity of the number of mixture components in the hierarchical Bayesian model. In Example 1, when the number of mixture components is 2, a minimum value of the WBIC is acquired.

Then, a path search is performed using the derived hierarchical Bayesian model. A path search is performed by selecting 5 high-ranking variables of the variable importance level from among explanatory variables of 10 types as intervention variables and fixing the remaining variables of 5 types. A unit for changing the intervention variables was set to 0.2σ in a training data set. For each instance, searches of a search repetition number L = 20,000 were performed, and a path having a value of the future progression degree of diabetes to be the lowest was acquired as an optimal path.

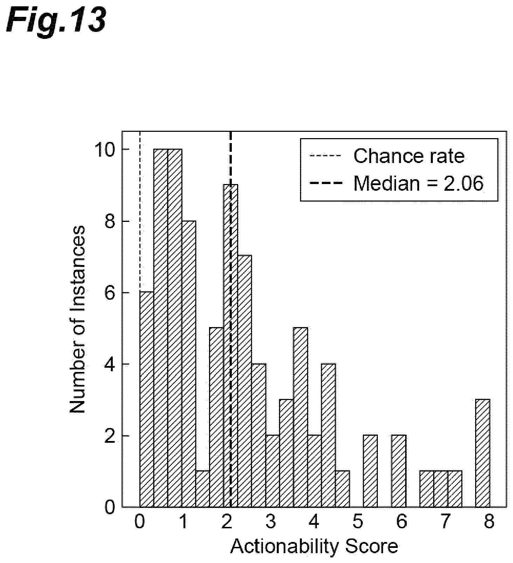

FIG. 13 is a histogram illustrating an actionability score of each instance in Example 1. In 83 instances among 87 instances, the actionability score is 0 or more, and a median value thereof is 2.06. This result represents that, even when an objective variable after improvement is the same, an actionability until reach thereto is different for each path. In addition, the result represents that most of paths retrieved by the health improvement path search device 1 have actionabilities higher than that of a random path.

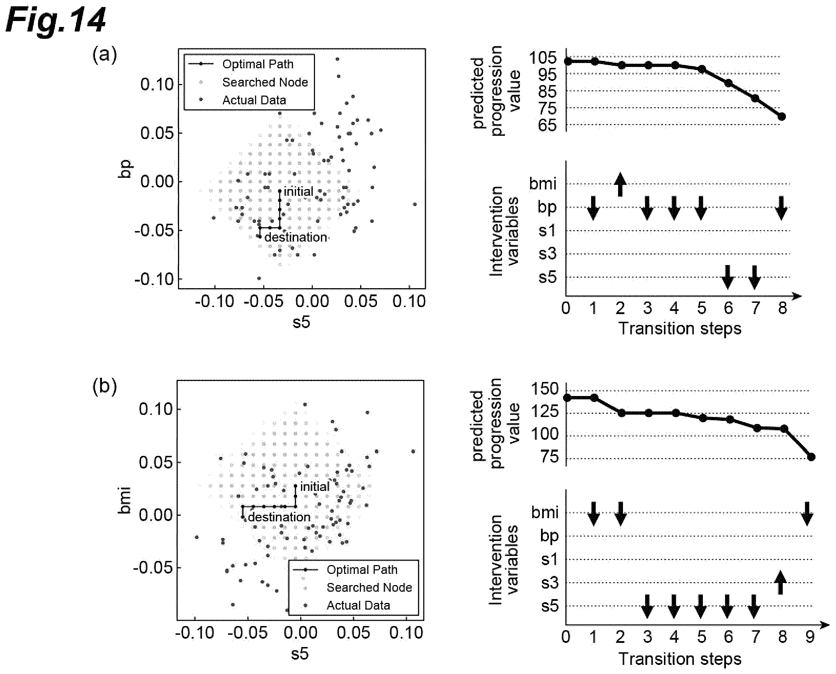

FIG. 14 is a diagram illustrating an example of a path search result in Example 1. FIGS. 14(a) and 14(b) illustrate examples of path search results for different instances. In each of FIGS. 14(a) and 14(b), a graph on the left side illustrates an example of an optimal path from a start point (initial) of a path search result to an end point (destination) thereof. The optimal path runs by way of nodes at which a probability of presence of actual data is high. In each of FIGS. 14(a) and 14(b), a graph on the right side represents an improvement value of a health index in the optimal path and a sequence of improving intervention variables. In the example of FIG. 14(a), an improvement sequence of bp, bmi, bp, s5, and bp is a path having a high actionability for improvement of diabetes, in other words, for improvement of a future advancement degree of diabetes that is a health index. In the example of FIG. 14(b), an improvement sequence of bmi, s5, s3, and bmi is a path having a high actionability for improving diabetes.

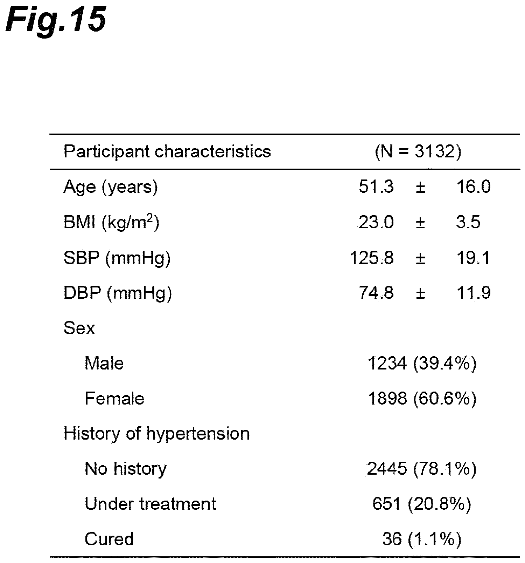

[Example 2] In Example 2, actionability was evaluated using a data set (hereinafter, referred to as an "IHPP data set") acquired using an Iwaki Health Promotion Project (hereinafter, referred to as "IHPP"; UMIN test ID: UMIN000040459). In the IHPP, for residents of 20 years old or more in Iwaki district, Hirosaki-shi, Aomori in Japan, broad health examination data such as biological/biochemical data, individual lifestyle action data, social environment data, and the like has been acquired from 2005. FIG. 15 illustrates an example of a data set in Example 2. In an IHPP data set, age, a BMI, a Systolic Blood Pressure (hereinafter, referred to as "SBP"), a Diastolic blood Pressure, gender, and a clinical history of hypertension are included. In Example 2, a scenario for improving the systolic blood pressure (SBP) was reviewed.

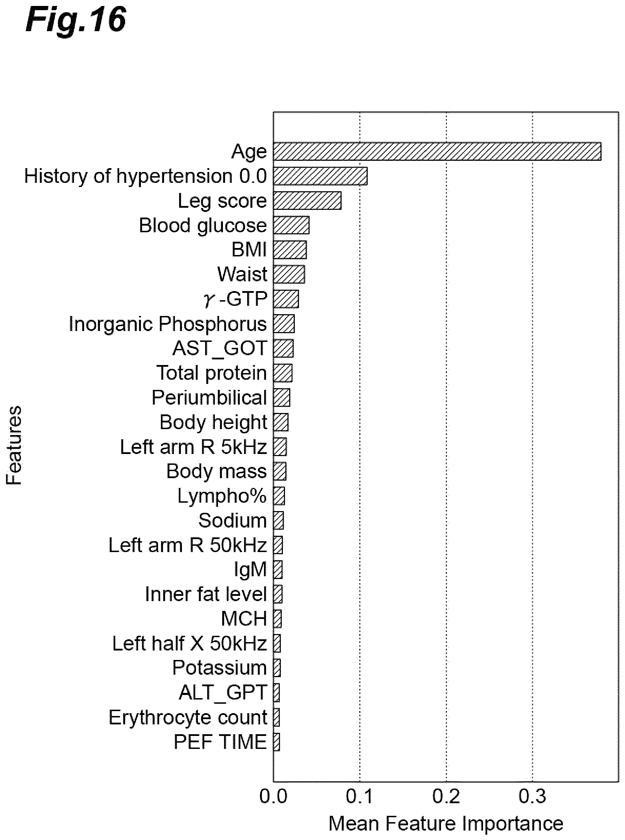

In the IHPP data set, measurement items and questionnaire answer items of 2,000 or more are included, and items having many missing values are included, and thus selection of variables was performed. More specifically, by excluding measurement items relating to a blood pressure, items relating to questionnaire answers, items including 25% or more missing values, and the like from explanatory variables, selection of variables was performed. In addition, by performing Recursive Feature Elimination (RFE) based on the XGBoost, explanatory variables are reduced. In data used for RFE, one hot encoding is applied to category variables, and missing values are substituted with a median value. In accordance with such a process, variables were reduced until 25 types of variables remained. FIG. 16 is a diagram illustrating variable importance levels of a regression model in Example 2. High-ranking items of the variable importance level are items relating to hypertension such as age, a leg score (a leg part muscle quantity score), a BMI, a waist, serum/blood sugar, γ-GTP, and the like, and, as a prediction model of a systolic blood pressure, it can be assumed that valid explanatory variables are selected at a clinical time point.

In Example 2, by randomly dividing an IHPP data set at a ratio of 80% and 20%, a regression model was generated using XGBoost with respective divided parts set as a training data set and a test data set. In Example 2, after substitution of missing values using a multiple imputation method, a regression model was generated. In estimation of missing values of the multiple imputation method, Bayesian Ridge was used for continuous variables, and Random Forest was used for discrete variables.

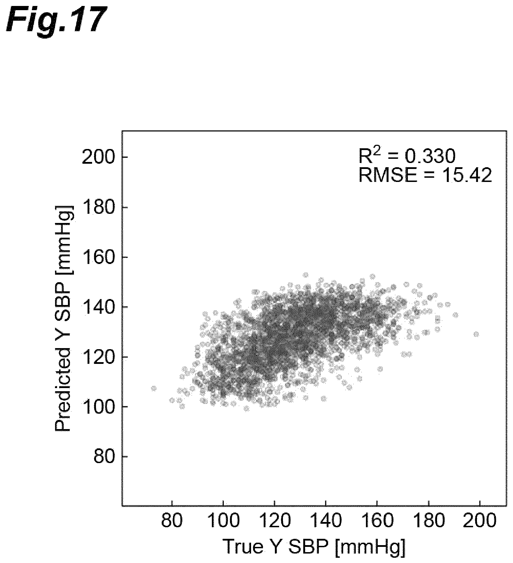

FIG. 17 is a diagram illustrating a score of a regression model in Example 2. In FIG. 17, a horizontal axis represents a true objective variable, and a vertical axis represents a predicted value of an objective variable using the regression model. In the generated regression model, an RMSE of a test data set was 15.42, and R² was 0.330.

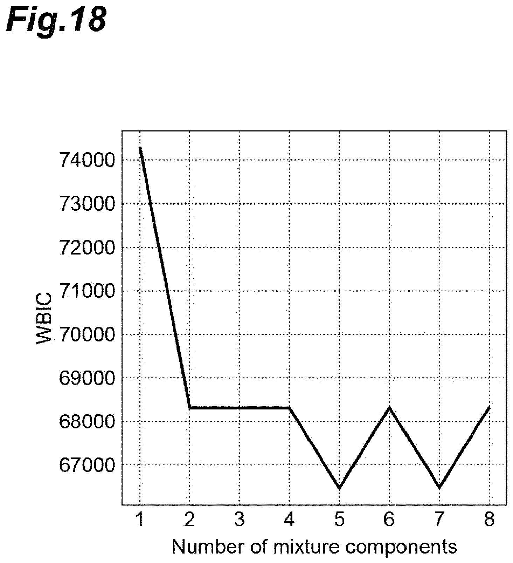

Next, on the basis of the IHPP data set and the predicted value of the regression model, a hierarchical Bayesian model was derived using hierarchical Bayesian modeling. FIG. 18 is a diagram illustrating an evaluation result of WBIC in Example 2. In FIG. 18, a horizontal axis represents the number of mixture components, and a vertical axis represents a value of the WBIC. In Example 2, a minimum value of the WBIC was acquired when the number of mixture components is 5.

Then, a path search was performed using the derived hierarchical Bayesian model. From among explanatory variables of 25 types, high-ranking five variables of the variable importance level described above, that is, the leg score, the serum/blood sugar, the BMI, the waist, and the γ-GTP were selected as intervention variables. A unit for changing an intervention variable was set to 0.2σ in the training data set. Relating to the systolic blood pressure, a scenario for lowering the blood pressure of a participant having a high value is assumed, and participant data in which the predicted systolic blood pressure is equal to or higher than mean+1σ of the training data set, and the intervention variables are not missing was set as instances of analysis targets. The number of instances that are analysis targets was 391. For each instance, searches of a search repetition number L = 20,000 were performed, and a path having the lowest systolic blood pressure value was acquired as an optimal path.

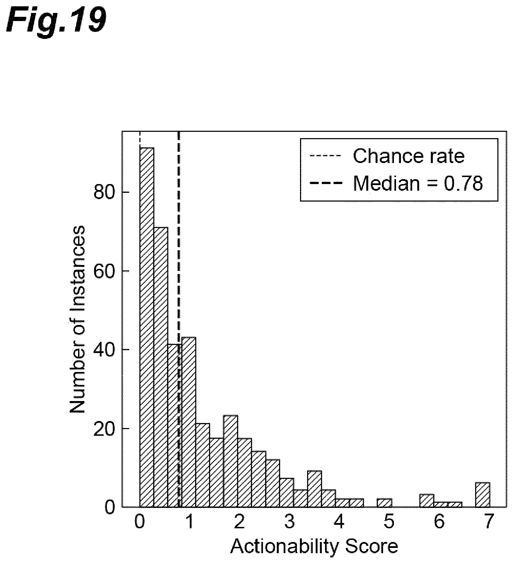

FIG. 19 is a histogram illustrating an actionability score of each instance in Example 2. The actionability score was 0 or more in 341 instances among 391 instances, and a median value was 0.78. This result represents that a path that is actionable for improvement of a systolic blood pressure is searchable using a data set acquired in an actual health examination, and it represents that most of paths retrieved by the health improvement path search device 1 have actionabilities higher than that of a random path.

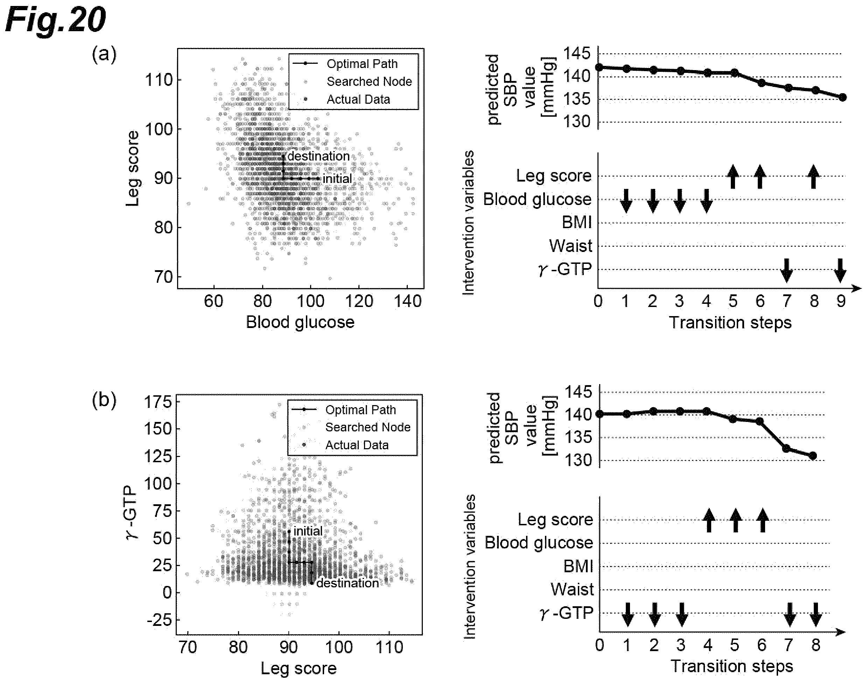

FIG. 20 is a diagram illustrating an example of a path search result in Example 2. FIGS. 20(a) and 20(b) illustrate examples of path search results for different instances. In each of FIGS. 20(a) and 20(b), a graph on a left side represents an example of an optimal path. The optimal path goes by way of nodes at which probabilities of presence of actual data are high. In each of FIGS. 20(a) and 20(b), a graph on a right side represents an improved value of a health index and an improvement sequence of intervention variables in the optimal path. In the example illustrated in FIG. 20(a), as a whole, an improvement sequence of the serum/blood sugar, the leg score, and the γ-GTP is a path having a high actionability for improvement of the systolic blood pressure that is a value of the health index. Such variables are associated with each other, and the path is valid as a path in which a plurality of variables change for improvement of a blood pressure. Also at a clinical time point for improvement of a blood pressure, a direction of changes of such variables is valid. For example, it is reported that high-priced serum/blood sugar is a risk factor of hypertension. In the example illustrated in FIG. 20(b), an improvement sequence of γ-GTP, the leg score, and γ-GTP is a path having a high actionability. Similar to FIG. 20(a), although a direction of changes of values of intervention variables at a clinical time point is valid, in the example illustrated in FIG. 20(b), it goes by way of nodes at which a predicted value of the regression model is temporarily higher than the original predicted value. The reason for this is that a node of which a predicted value is the best is selected, and a probabilistic optimal path until reach of the node is acquired, and predicted values of nodes gone through are not considered. The health improvement path search device 1 may exclude a node of which a predicted value is degraded from a search range.

[Operation and effect] Next, operations and effects of the health improvement path search device 1 according to this embodiment will be described.

A health improvement path search device 1 according to this embodiment includes: a first model generating unit 12 configured to generate a first model predicting a value of a health index that is an objective variable on the basis of values of a plurality of explanatory variables; a second model generating unit 13 configured to generate a second model deriving a probability of taking a state calculated using the first model representing a degree of easiness of presence of each of combinations of the values of the plurality of explanatory variables input to the first model and the value of the health index predicted using the first model on the basis of the values of the plurality of explanatory variables; and a path searching unit 14 configured to divide a variable space of the plurality of explanatory variables into a lattice, and in a graph with lattice points as nodes, to derive the value of the health index and the probability corresponding to each node on the basis of the first model and the second model with a plurality of nodes within a predetermined range from current values of the plurality of explanatory variables set as inputs, specify a plurality of paths transitioning to each of the nodes from the current values as start points for transitioning between the values of the plurality of explanatory variables of continuous values, specify one or a plurality of paths in which the value of the health index at an end point is improved from the current value of the health index among the plurality of paths as candidate paths, and specify a path for which a product of the probabilities of the nodes included in the path is a maximum among the candidate paths as a health improvement path; wherein variables refer to measured values of a person measured in a health examination or the like, explanatory variables are variables that become causes of a causal relationship, and objective variables are variables that become a result of the causal relationship; and wherein the health improvement path is an improvement sequence for measurement values of a person that is performed for improving values of health indexes.

A health improvement path search method according to this embodiment includes: a step of generating a first model (S1) predicting a value of a health index that is an objective variable on the basis of values of a plurality of explanatory variables; a step of generating a second model (S2) deriving a probability representing a degree of easiness of presence of each of combinations of the values of the plurality of explanatory variables input to the first model and the value of the health index predicted using the first model on the basis of the values of the plurality of explanatory variables; and a step (S3) of divide a variable space of the plurality of explanatory variables into a lattice, and in a graph with lattice points as nodes, and deriving the value of the health index and the probability corresponding to node on the basis of the first model and the second model with a plurality of nodes within a predetermined range from current values of the plurality of explanatory variables set as inputs, specifying a plurality of paths transitioning to each of the measurement target values from the current values as start points for transitioning between the values of the plurality of explanatory variables of continuous values, specifying one or a plurality of paths in which the value of the health index at an end point is improved from the current value of the health index among the plurality of paths as candidate paths, and specifying a path for which a product of the probabilities of the nodes included in the path is a maximum among the candidate paths as a health improvement path; wherein variables refer to measured values of a person measured in a health examination or the like, explanatory variables are variables that become causes of a causal relationship, and objective variables are variables that become a result of the causal relationship; and wherein the health improvement path is an improvement sequence for measurement values of a person that is performed for improving values of health indexes.

In the health improvement path search device 1 and the health improvement path search method according to this embodiment, a first model predicting a health index that is an objective variable on the basis of a plurality of explanatory variables is generated, and a second model deriving a presence probability of each of combinations of input values of the plurality of explanatory variables and a value of a health index that is a predicted value thereof is generated. When a plurality of measurement target values are input to the first model and the second model, a presence probability for each combination of each measurement target value and a value of the health index that is a predicted value thereof is derived. Then, in this health improvement path search device 1 and this health improvement path search method, among a plurality of paths transitioning from current values of the plurality of explanatory variables as a start point to each measurement target value, a path for which the value of the health index at an end point is improved from the current value of the health index, and a product of presence probabilities of the measurement target values within the path is a maximum is specified as a health improvement path. According to such a configuration, when current values of a plurality of explanatory variables acquired through a health examination or the like are input, from a plurality of measurement target values within a predetermined range from a current value, a presence probability of a combination of each measurement target value and a value of a health index that is a predicted value thereof is derived. Then, among paths transitioning between the measurement target values, a path for which a health index is improved from a start point to an end point, and a presence probability of each measurement target value within the path is a maximum is specified. The path specified in this way becomes a path that represents a sequence for going by way of only realistic values for transition to each measurement target value having a high presence probability until the health index is improved and specifically improves the health. By illustrating such a health improvement path, a health improvement process that can be performed by a person can be proposed.

In the health improvement path search device 1 described above, the path searching unit 14 may specify a path for which the value of the health index is improved the most as the candidate path among paths for which the value of the health index is improved from the current value of the health index. In accordance with this, a health improvement process expected to have the best result of improvement of the health index can be proposed.

The path searching unit 14, after a first process of selecting the values of the plurality of explanatory variables that are approximated to reference values as measurement target values with the current values set as the reference values is performed, may repeatedly perform a second process of selecting the values of the plurality of explanatory variables that are approximated to the reference value as the measurement target values with the measurement target value of which the presence probability at the time of being input to the second model is the highest among the selected measurement target values set as the new reference value. By avoiding a path transitioning between measurement target values of which presence probabilities are low among input measurement target values, a realistic path can be efficiently retrieved.

In the health improvement path search device 1 described above, the path searching unit 14 may specify a shortest path randomly transitioning between the measurement target values from a start point to an end point as a random path and specify a path for which a product of presence probabilities of measurement target values included in the path is a maximum and is equal to or larger than a product of presence probabilities of measurement target values included in the random path as the health improvement path among the candidate paths. A health improvement path having a product of presence probabilities that is equal to or higher than that of a random path is specified, and a degree of efficiency of the health improvement path specified on the basis of the product of presence probabilities with respect to a random path can be represented, and a health improvement process that can be performed by a person can be proposed.

As above, although the health improvement path search device 1 according to this embodiment has been described, the present invention is not limited to the embodiment described above. Although among paths for which the value of the health index is improved from the current value of the health index, a path for which the value of the health index is improved the most has been described to be specified as a candidate path, the path searching unit 14 may specify a path for which the value of the health index coincides with a target value of the health index set in advance as the candidate path among paths for which the value of the health index is improved from the current value of the health index. For example, the path searching unit 14 may apply a search condition that a search ends in a case in which a predicted value coincides with or is below (or above) a target value or the like. In accordance with this, also in a case in which a target value of a health index is set in advance through a guideline of the health index, a clinical knowledge, or the like, a path can be retrieved while the search cost is reduced.

In addition, in the embodiment, although an example in which the first model generating unit 12 uses XGBoost has been described, the present invention does not depend on the model, and thus a regression model of deep learning or the like may be used. Although explanatory variables in a hierarchical Bayesian model have been described by assuming a normal distribution or a category distribution, a distribution according to data may be selected. In accordance with this, data having much noise, data having much missing, and the like that can be frequently seen in medical data also can be handled. Although the path searching unit 14 performs a path search by changing intervention variables in a specific unit, for variables that can be measured more accurately, the unit of change may be adjusted in accordance with a user's taste or environments. In addition, in the embodiment, although intervention variables are selected from those of high-ranking variable importance levels, intervention points may be retrieved in combination with a technology such as Counterfactuals in explainable AI (XAI). In addition, in the embodiment, although an example in which a hierarchical Bayesian model is generated as the second model has been described, the second model is not limited thereto, and an arbitrary model that can express a presence probability of data can be used as the second model.

### Reference Signs List

1 Health improvement path search device
11 Database
12 First model generating unit
13 Second model generating unit
14 Path searching unit

## Claims

1. A health improvement path search device comprising:
a first model generating unit (12) configured to generate a first model predicting a value of a health index that is an objective variable on the basis of values of a plurality of explanatory variables;
a second model generating unit (13) configured to generate a second model deriving a probability of taking a state calculated using the first model representing a degree of easiness of presence of each of combinations of the values of the plurality of explanatory variables input to the first model and the value of the health index predicted using the first model on the basis of the values of the plurality of explanatory variables; and
a path searching unit (14) configured to divide a variable space of the plurality of explanatory variables into a lattice, and in a graph with lattice points as nodes, to derive the value of the health index and the probability corresponding to each node on the basis of the first model and the second model with a plurality of nodes within a predetermined range from current values of the plurality of explanatory variables set as inputs, specify a plurality of paths transitioning to each of the nodes from the current values as start points for transitioning between the values of the plurality of explanatory variables of continuous values, specify one or a plurality of paths in which the value of the health index at an end point is improved from the current value of the health index among the plurality of paths as candidate paths, and specify a path for which a product of the probabilities of the nodes included in the path is a maximum among the candidate paths as a health improvement path;
wherein variables refer to measured values of a person measured in a health examination or the like, explanatory variables are variables that become causes of a causal relationship, and objective variables are variables that become a result of the causal relationship; and
wherein the health improvement path is an improvement sequence for measurement values of a person that is performed for improving values of health indexes.

2. The health improvement path search device according to claim 1, wherein the path searching unit specifies a path for which the value of the health index is improved the most as the candidate path among paths for which the value of the health index is improved from the current value of the health index.

3. The health improvement path search device according to claim 1 or 2, wherein the path searching unit specifies a path for which the value of the health index coincides with a target value of the health index set in advance as the candidate path among paths for which the value of the health index is improved from the current value of the health index.

4. The health improvement path search device according to any one of claims 1 to 3, wherein, after a first process of selecting the values of the plurality of explanatory variables that are approximated to reference values as nodes with the current values set as the reference values is performed, the path searching unit repeatedly performs a second process of selecting the values of the plurality of explanatory variables that are approximated to the reference value as the nodes with the node of which the presence probability at the time of being input to the second model is the highest among the nodes set as the new reference value.

5. The health improvement path search device according to any one of claims 1 to 4, wherein the path searching unit specifies a shortest path randomly transitioning between the nodes from a start point to an end point as a random path and specifies a path for which a product of probabilities of nodes included in the path is a maximum and is equal to or larger than a product of probabilities of nodes included in the random path as the health improvement path among the candidate paths.

6. A health improvement path search method performed by an information processing device, the health improvement path search method comprising:
a step of generating a first model (S1) predicting a value of a health index that is an objective variable on the basis of values of a plurality of explanatory variables;
a step of generating a second model (S2) deriving a probability of taking a state calculated using the first model representing a degree of easiness of presence of each of combinations of the values of the plurality of explanatory variables input to the first model and the value of the health index predicted using the first model on the basis of the values of the plurality of explanatory variables; and
a step (S3) of divide a variable space of the plurality of explanatory variables into a lattice, and in a graph with lattice points as nodes, and deriving the value of the health index and the probability corresponding to node on the basis of the first model and the second model with a plurality of nodes within a predetermined range from current values of the plurality of explanatory variables set as inputs, specifying a plurality of paths transitioning to each of the measurement target values from the current values as start points for transitioning between the values of the plurality of explanatory variables of continuous values, specifying one or a plurality of paths in which the value of the health index at an end point is improved from the current value of the health index among the plurality of paths as candidate paths, and specifying a path for which a product of the probabilities of the nodes included in the path is a maximum among the candidate paths as a health improvement path;
wherein variables refer to measured values of a person measured in a health examination or the like, explanatory variables are variables that become causes of a causal relationship, and objective variables are variables that become a result of the causal relationship; and
wherein the health improvement path is an improvement sequence for measurement values of a person that is performed for improving values of health indexes.

## Patentansprüche

1. Vorrichtung zur Ermittlung eines Gesundheitsverbesserungspfads, umfassend:
eine erste Modellgenerierungseinheit (12), die derart konfiguriert ist, ein erstes Modell zur Vorhersage eines Wertes eines Gesundheitsindexes, der eine objektive Variable ist, auf der Grundlage von Werten einer Vielzahl von erklärenden Variablen zu erzeugen;
eine zweite Modellgenerierungseinheit (13), die derart konfiguriert ist, ein zweites Modell zur Ableitung einer Aufenthaltswahrscheinlichkeit zu erzeugen , mit der ein Zustand eintreten wird, der mittels des ersten Modells berechnet wurde und einem Maß der Leichtigkeit des Vorhandenseins jeder Kombination der Werte der Vielzahl von erklärenden Variablen, welche in das erste Modell eingegeben wurden, und des Wertes des Gesundheitsindexes darstellt, der mittels des ersten Modells auf der Grundlage der Werte der mehreren erklärenden Variablen vorhergesagt wurde; und
eine Pfadsucheinheit (14), die derart konfiguriert ist, dass sie einen Variablenraum der Vielzahl von erklärenden Variablen in ein Gitter und in einen Graph mit Gitterpunkten als Knoten unterteilt, um den Wert des Gesundheitsindex und die Wahrscheinlichkeit, die jedem Knoten entspricht, auf der Grundlage des ersten Modells und des zweiten Modells mit einer Vielzahl von Knoten in einem vorbestimmten Bereich aus aktuellen Werten der Vielzahl von erklärenden Variablen, die als Eingaben definiert sind, abzuleiten,
um eine Vielzahl von Pfaden anzugeben, die einen Übergang zu jedem der Knoten ausgehend von den aktuellen Werten als Ausgangspunkte für einen Übergang zwischen den Werten der Vielzahl von erklärenden Variablen mit kontinuierlichen Werten bewirken,
um einen Pfad oder eine Vielzahl von Pfaden festzulegen, in denen der Wert des Gesundheitsindex an einem Endpunkt ausgehend von dem aktuellen Wert des Gesundheitsindex unter der Vielzahl von Pfaden als Kandidatenpfaden verbessert wird,
und um einen Pfad festzulegen, für den ein Produkt der Wahrscheinlichkeiten der in dem Pfad enthaltenen Knoten auf einem maximalen Niveau unter den Kandidatenpfaden liegt, als Gesundheitsverbesserungspfad;
wobei sich Variablen auf Messwerte einer Person beziehen, die bei einer Gesundheitsuntersuchung oder dergleichen gemessen wurden, wobei erklärende Variablen solche Variablen sind, die Ursachen in einer Ursache-Wirkungs-Beziehung werden, und objektive Variablen solche Variablen sind, die eine Wirkung der Ursache-Wirkungs-Beziehung werden; und
wobei der Gesundheitsverbesserungspfadeine Verbesserungssequenz für Messwerte einer Person ist, die zur Verbesserung von Gesundheitsindexwerten durchgeführt wird.

2. Vorrichtung zur Suche eines Gesundheitsverbesserungspfads gemäß Anspruch 1, wobei die Pfadsucheinheit einen Pfad, bei dem der Wert des Gesundheitsindex am stärksten verbessert wird, als Kandidatenpfad unter den Pfaden, bei denen der Wert des Gesundheitsindex gegenüber dem aktuellen Wert des Gesundheitsindex verbessert wird, angibt.

3. Vorrichtung zur Suche eines Gesundheitsverbesserungspfads gemäß Anspruch 1 oder 2, wobei die Pfadsucheinheit einen Pfad, bei dem der Wert des Gesundheitsindexes mit einem vorab festgelegten Zielwert des Gesundheitsindexes übereinstimmt, als Kandidatenpfad unter den Pfaden ermittelt, bei denen der Wert des Gesundheitsindexes gegenüber dem aktuellen Wert des Gesundheitsindexes verbessert ist.

4. Vorrichtung zur Suche eines Gesundheitsverbesserungspfads gemäß einem der Ansprüche 1 bis 3, wobei nach einem ersten Prozess zur Auswahl der Werte der Vielzahl von erklärenden Variablen, die sich Referenzwerten als Knoten mit den aktuellen Werten annähern, die als Referenzwerte definiert sind, die Pfadsucheinheit wiederholt einen zweiten Prozess zur Auswahl der Werte der mehreren erklärenden Variablen durchführt, die sich dem Referenzwert als Knoten mit dem Knoten annähern, dessen Existenzwahrscheinlichkeit zum Zeitpunkt der Eingabe in das zweite Modell unter den als neuer Referenzwert definierten Knoten am höchsten ist.

5. Vorrichtung zur Suche eines Gesundheitsverbesserungspfads gemäß einem der Ansprüche 1 bis 4, wobei die Pfadsucheinheit einen kürzesten Pfad, der einen zufälligen Übergang zwischen den Knoten von einem Startpunkt zu einem Endpunkt vollzieht, als zufälligen Pfad angibt, und einen Pfad, bei dem ein Produkt aus den Wahrscheinlichkeiten der in dem Pfad enthaltenen Knoten auf einem maximalen Niveau liegt und gleich oder größer ist als ein Produkt aus den Wahrscheinlichkeiten der in dem zufälligen Pfad enthaltenen Knoten, als Pfad zur Verbesserung der Gesundheit unter den Kandidatenpfaden angibt.

6. Verfahren zur Suche eines Gesundheitsverbesserungspfads, das von einer Informationsverarbeitungsvorrichtung durchgeführt wird, wobei das Verfahren zur Suche eines Gesundheitsverbesserungspfads umfasst:
einen Schritt zum Erzeugen eines ersten Modells (S1) zum Vorhersagen eines Wertes eines Gesundheitsindexes, der eine objektive Variable ist, auf der Grundlage von Werten einer Vielzahl von erklärenden Variablen;
einen Schritt zum Erzeugen eines zweiten Modells (S2) zum Ableiten einer Aufenthaltswahrscheinlichkeit für das Eintreten eines Zustands, der mittels des ersten Modells berechnet wurde und einem Maß Grad der Leichtigkeit des Vorhandenseins jeder Kombination der Werte der mehreren erklärenden Variablen, welche in das erste Modell eingegeben wurden, und des Wertes des Gesundheitsindexes darstellt, der mittels des ersten Modells auf der Grundlage der Werte der Vielzahl von erklärenden Variablen vorhergesagt wurde; und
einen Schritt (S3) zum Aufteilen eines Variablenraums der Vielzahl von erklärenden Variablen in ein Gitter und in einen Graph mit Gitterpunkten als Knoten, und Ableiten des Gesundheitsindexwerts und der Wahrscheinlichkeit, die einem Knoten entsprechen, auf der Grundlage des ersten Modells und des zweiten Modells mit einer Vielzahl von Knoten in einem vorbestimmten Bereich aus aktuellen Werten der Vielzahl von erklärenden Variablen, die als Eingaben definiert sind,
zum Festlegen einer Vielzahl von Pfaden, die einen Übergang zu jedem der Zielmesswerte von den aktuellen Werten als Ausgangspunkte für einen Übergang zwischen den Werten der Vielzahl von erklärenden Variablen mit kontinuierlichen Werten bewirken, zum Festlegen eines Pfades oder einer Vielzahl von Pfaden, in denen der Wert des Gesundheitsindexes an einem Endpunkt ausgehend vom aktuellen Wert des Gesundheitsindexes unter den Pfaden als Kandidatenpfade verbessert wird, und Festlegen eines Pfades, für den ein Produkt der Wahrscheinlichkeiten der in dem Pfad enthaltenen Knoten unter den Kandidatenpfaden ein maximales Niveau aufweist, als Gesundheitsverbesserungspfad;
wobei Variablen sich auf Messwerte einer Person beziehen, die bei einer Gesundheitsuntersuchung oder dergleichen gemessen wurden, wobei erklärende Variablen solche Variablen sind, die Ursachen in einer Ursache-Wirkungs-Beziehung werden, und objektive Variablen solche Variablen sind, die eine Wirkung der Ursache-Wirkungs-Beziehung werden; und
wobei der Gesundheitsverbesserungspfadeine Verbesserungssequenz für Messwerte einer Person ist, die zur Verbesserung von Gesundheitsindexwerten durchgeführt wird.

## Revendications

1. Dispositif de recherche de voie d'amélioration de santé comprenant :
une première unité de génération de modèle (12) configurée pour générer un premier modèle de prédiction d'une valeur d'un indice de santé qui est une variable objective sur la base de valeurs d'une pluralité de variables explicatives ;
une seconde unité de génération de modèle (13) configurée pour générer un second modèle de dérivation d'une probabilité de prendre un état calculé au moyen du premier modèle représentant un degré de facilité d'existence de chacune de combinaisons des valeurs de la pluralité de variables explicatives entrées dans le premier modèle et de la valeur de l'indice de santé prédit au moyen du premier modèle sur la base des valeurs de la pluralité de variables explicatives ; et
une unité de recherche de voie (14) configurée pour diviser un espace de variable de la pluralité de variables explicatives en un réseau, et dans un graphique avec des points de réseau en tant que nœuds, pour dériver la valeur de l'indice de santé et la probabilité correspondant à chaque nœud sur la base du premier modèle et du second modèle avec une pluralité de nœuds dans une plage prédéterminée à partir de valeurs actuelles de la pluralité de variables explicatives définies en tant qu'entrées, spécifier une pluralité de voies effectuant une transition vers chacun des nœuds à partir des valeurs actuelles en tant que points de départ pour effectuer une transition entre les valeurs de la pluralité de variables explicatives de valeurs continues, spécifier une voie ou une pluralité de voies dans laquelle la valeur de l'indice de santé à un point final est améliorée à partir de la valeur actuelle de l'indice de santé parmi la pluralité de voies en tant que voies candidates, et spécifier une voie pour laquelle un produit des probabilités des nœuds inclus dans la voie est à un niveau maximum parmi les voies candidates en tant que voie d'amélioration de santé ;
dans lequel des variables se réfèrent à des valeurs mesurées d'une personne mesurées lors d'un examen de santé ou similaire, des variables explicatives sont des variables qui deviennent des causes d'une relation de cause à effet, et des variables objectives sont des variables qui deviennent un résultat de la relation de cause à effet ; et
dans lequel la voie d'amélioration de santé est une séquence d'amélioration pour des valeurs de mesure d'une personne qui est réalisée pour améliorer des valeurs d'indices de santé.

2. Dispositif de recherche de voie d'amélioration de santé selon la revendication 1, dans lequel l'unité de recherche de voie spécifie une voie pour laquelle la valeur de l'indice de santé est la plus améliorée en tant que voie candidate parmi des voies pour lesquelles la valeur de l'indice de santé est améliorée à partir de la valeur actuelle de l'indice de santé.

3. Dispositif de recherche de voie d'amélioration de santé selon la revendication 1 ou 2, dans lequel l'unité de recherche de voie spécifie une voie pour laquelle la valeur de l'indice de santé coïncide avec une valeur cible de l'indice de santé définie à l'avance en tant que voie candidate parmi des voies pour lesquelles la valeur de l'indice de santé est améliorée à partir de la valeur actuelle de l'indice de santé.

4. Dispositif de recherche de voie d'amélioration de santé selon l'une quelconque des revendications 1 à 3, dans lequel, après qu'un premier processus de sélection des valeurs de la pluralité de variables explicatives qui s'approchent de valeurs de référence en tant que nœuds avec les valeurs actuelles définies en tant que valeurs de référence est réalisé, l'unité de recherche de mode réalise de façon répétée un second processus de sélection des valeurs de la pluralité de variables explicatives qui s'approchent de la valeur de référence en tant que nœuds avec le nœud dont la probabilité d'existence au moment de son entrée dans le second modèle est la plus élevée parmi les nœuds défini en tant que nouvelle valeur de référence.

5. Dispositif de recherche de voie d'amélioration de santé selon l'une quelconque des revendications 1 à 4, dans lequel l'unité de recherche de voie spécifie une voie la plus courte effectuant une transition aléatoire entre les nœuds à partir d'un point de départ jusqu'à un point final en tant que voie aléatoire et spécifie une voie pour laquelle un produit de probabilités de nœuds inclus dans la voie est à un niveau maximum et est égal ou supérieur à un produit de probabilités de nœuds inclus dans la voie aléatoire en tant que voie d'amélioration de santé parmi les voies candidates.

6. Procédé de recherche de voie d'amélioration de santé réalisé par un dispositif de traitement d'informations, le procédé de recherche de voie d'amélioration de santé comprenant :
une étape de génération d'un premier modèle (S1) de prédiction d'une valeur d'un indice de santé qui est une variable objective sur la base de valeurs d'une pluralité de variables explicatives ;
une étape de génération d'un second modèle (S2) de dérivation d'une probabilité de prendre un état calculé au moyen du premier modèle représentant un degré de facilité d'existence de chacune de combinaisons des valeurs de la pluralité de variables explicatives entrées dans le premier modèle et de la valeur de l'indice de santé prédit au moyen du premier modèle sur la base des valeurs de la pluralité de variables explicatives ; et
une étape (S3) de division d'un espace de variable de la pluralité de variables explicatives en un réseau, et dans un graphique avec des points de réseau en tant que nœuds, et de dérivation de la valeur de l'indice de santé et de la probabilité correspondant à un nœud sur la base du premier modèle et du second modèle avec une pluralité de nœuds dans une plage prédéterminée à partir de valeurs actuelles de la pluralité de variables explicatives définies en tant qu'entrées, de spécification d'une pluralité de voies effectuant une transition vers chacune des valeurs cibles de mesure à partir des valeurs actuelles en tant que points de départ pour effectuer une transition entre les valeurs de la pluralité de variables explicatives de valeurs continues, de spécification d'une voie ou d'une pluralité de voies dans laquelle la valeur de l'indice de santé à un point final est améliorée à partir de la valeur actuelle de l'indice de santé parmi la pluralité de voies en tant que voies candidates, et de spécification d'une voie pour laquelle un produit des probabilités des nœuds inclus dans la voie est à un niveau maximum parmi les voies candidates en tant que voie d'amélioration de santé ;
dans lequel des variables se réfèrent à des valeurs mesurées d'une personne mesurées lors d'un examen de santé ou similaire, des variables explicatives sont des variables qui deviennent des causes d'une relation de cause à effet, et des variables objectives sont des variables qui deviennent un résultat de la relation de cause à effet ; et
dans lequel la voie d'amélioration de santé est une séquence d'amélioration pour des valeurs de mesure d'une personne qui est réalisée pour améliorer des valeurs d'indices de santé.
